# EUROPEAN PATENT APPLICATION

(11) **EP 2 140 894 A1**
(43) Date of publication of application: **06.01.2010**
(21) Application number: 08159341.0
(22) Date of filing: 30.06.2008
(51) Int. Cl.: A61M 5/142

(54) **Infusion device with optical status indicators**

(71) Applicant: F.HOFFMANN-LA ROCHE AG, 4070 Basel (CH); Roche Diagnostics GmbH, 68305 Mannheim (DE)
(72) Inventor: Jeanbourquin, Edgar, 3075, Rüfenacht (CH)
(74) Representative: Schwabe - Sandmair - Marx

(57) **Abstract**

Infusion device with a housing, an actuator and a control unit, characterized by a plurality of optical indicators in a defined geometrical arrangement for indicating the state of the infusion pump device.

## Description

The present invention relates to an infusion device, for example an insulin pump, with a housing, an actuator and a control unit.

The ongoing miniaturisation of extracorporeal insulin pumps and similar medical devices for administering drugs to a patient makes it more and more difficult to provide a display device which is capable of showing the information necessary for a device operation, error and alert information, and the like. Even though the systems may largely rely on a remote user interface such as a PDA like device, displaying different kinds of information on the device itself is desirable or required by national law.

Current insulin pumps use LCDs or illuminated displays. Those displays are rather thick, have a flat surface, require clean and scratch-free windows in the housing, are susceptible to shock, expensive and need a considerable amount of energy.

It is an object of the present invention to provide an infusion device providing an easy to read way for indicating the state of the infusion pump.

This problem is solved by claim 1. Preferred embodiments are defined in the dependent claims.

The infusion device according to the present invention comprises a housing, an actuator and a control unit. The actuator, for example a motor, is used to deliver the substance or drug stored in the device to the patient, for example by moving a piston in the drug cartridge or operating a pump. The actuator may also be an electromagnetic actuator or a shape memory alloy actuator, for example.

The device is characterized by a plurality of optical indicators in a defined geometrical arrangement for indicating the state of the infusion device which can replace a display. This means that, in a first embodiment, the optical indicators are the only means for indicating the state of the infusion device and there is no other display. In another embodiment, the optical indicators are the main means for providing the state of the infusion device and are complemented by one or more displays, such as an LCD display.

This means that an optical indicator does not only indicate one special state of only one function of the infusion device by turning the indicator on or off, but the plurality of optical indicators can indicate the state by a geometrical and/or temporal coding. The state consists of several sub-states like remaining battery power, remaining drug or delivery rate.

The control unit is configured to control the optical indicators and, optionally, the operation of the actuator. The optical indicators may have any shape, for example circular, rectangular or quadratic. The optical indicators are preferably discrete, at least in part. This means that the indicators are separated from each other and are separate assemblies. The major advantage of using discrete optical indicators is that they can be easily placed even on uneven or curved surfaces of the housing.

Preferably, all optical indicators or some of the optical indicators are light emitting diodes (LEDs). LEDs are easily readable, even from patients being impaired in vision. The optical indicators may at least in part be implemented as surface mounted devices (SMDs). The optical indicators can also be bulbs or optical fibres. Optical fibres can be implemented in in-mould-labelling technology, for example using a light guiding sheet with symbols, text or dots being inserted in a tool and then injection moulded.

The optical indicators can be attached to a print, a carrier or a circuit board. The optical indicators can be placed in holes or recesses of the housing, thus further reducing the required height of the housing. The optical indicators can be inlayed, pressed, glued or welded into the housing. The electrical contact can be realized by wire, cable, flex print, spring or integrated conductors according to MID technology (Molded Interconnected Device).

In one embodiment of the invention, the housing is at least partially transparent, especially in places where the optical indicators are placed within the housing.

In a preferred embodiment, some or all of the optical indicators are multi-colour optical indicators. This allows for providing different information with the same optical indicators.

In one embodiment of the invention, some or all of the optical indicators are located on some or all edges and/or corners of the housing. This means that the optical indicators can be checked from a wide range of angles. In a preferred embodiment, at least one optical indicator is placed on each edge and/or corner of the housing or a plate, especially the front plate, or front side of the housing. The front plate or side of the housing is a main surface or plate that can contain keys or buttons to operate the device. It is usually opposite to the side of the housing that faces the user of the device or fixing means of the device. This arrangement of indicators further increases the range of angles from which the optical indicators can be read.

In another embodiment, all optical indicators are located on the edges and/or corners of the housing. This means that every optical indicator is in contact with at least two plates of the housing. In a special representation of this embodiment, at least one optical indicator is placed on each edge and/or corner of a plate of the housing, especially the front plate.

In another embodiment of the invention, at least some of the optical indicators are arranged in or on an ellipse. In a more specific embodiment, at least some of the optical indicators are arranged in two or more concentric ellipses. This arrangement of the optical indicators allows for an easy to read indication of the state of the infusion device. In one embodiment, an ellipse is constituted by at least five optical indicators, for example 5, 6, 7, 8, 9, 10, 11 or 12 optical indicators. Optionally, an optical indicator is arranged in the centre of the ellipse or ellipses. An ellipse may be a circle as a special embodiment of an ellipse. An ellipse (or circle) is preferably, but not necessarily a full ellipse (or circle). It can as well be an arc of an ellipse or circle. The LEDs on an ellipse or circle are preferably placed equally spaced.

In yet another embodiment of the invention, at least some of the optical indicators are arranged in a matrix. This enables another easy to read indication of the state of the infusion device. It is possible to combine the circular and matrix arrangement of the optical indicators, for example as a matrix of optical indicators surrounded by one or more circles of optical indicators.

In a preferred embodiment, the same information may be, at least in part, indicated by more than one optical indicator or more than one group of indicators, meaning a redundant indication. For example, optical indicators may be provided on several of the housing plates, with each information indicated on one, two or more housing plates, such that the optical indicators on at least one of the housing plates are easily visible in each wearing position of the infusion device. In an especially preferred embodiment, redundant optical indicators or redundant groups of optical indicators are provided to indicate information such as the general state of operation of the actuator, while optical indicators for additional or more detailed information may be provided only once, for example on the front plate of the housing. The additional information is the remaining cartridge content or an error or alert code, for example.

Optionally, the infusion device contains at least one sensor such as a tilt switch or an orientation switch to automatically determine which of the redundant optical indicators or groups of optical indicators should be used for the indication and which optical indicators should be switched off since they are not visible for the user. For example, only optical indicators pointing forwards and upwards with respect to the orientation of the user may be used for the indication, while optical indicators pointing backwards or downwards may be switched off in order to save energy.

The present invention is further described by some special embodiments illustrated by the accompanying drawings. The drawings show:
- Figure 1:: a schematic view of an infusion device,
- Figure 2:: LEDs fastened on a carrier,
- Figure 3:: LEDs fixed in the housing,
- Figure 4:: an infusion device with LEDs on edges and corners of the housing,
- Figure 5:: an arrangement of 2 LEDs,
- Figure 6:: 2 LED indication patterns,
- Figure 7:: an arrangement of 3 LEDs,
- Figure 8:: 3 LED indication patterns,
- Figure 9:: an arrangement of 4 LEDs,
- Figure 10:: 4 LED indication patterns,
- Figure 11:: a circular arrangement of LEDs,
- Figure 12:: symmetrical indication patterns using the arrangement of Figure 11,
- Figure 13:: alert indication patterns using the arrangement of Figure 11,
- Figure 14:: clock-like indication patterns using the arrangement of Figure 11,
- Figure 15:: two-colour indication patterns using the arrangement of Figure 11,
- Figure 16:: a two-circle arrangement of LEDs,
- Figure 17:: two circle indication patterns using the arrangement of Figure 16,
- Figure 18:: an elliptical arrangement of LEDs,
- Figure 19:: a curved arrangement of LEDs,
- Figure 20:: a dice arrangement of LEDs,
- Figure 21:: dice indication patterns using the arrangement of Figure 20.
- Figure 22:: a matrix and circular arrangement of LEDs,
- Figure 23:: matrix and circular indication patterns using the arrangement of Figure 22 and
- Figure 24:: 5x7 matrix indication patterns.

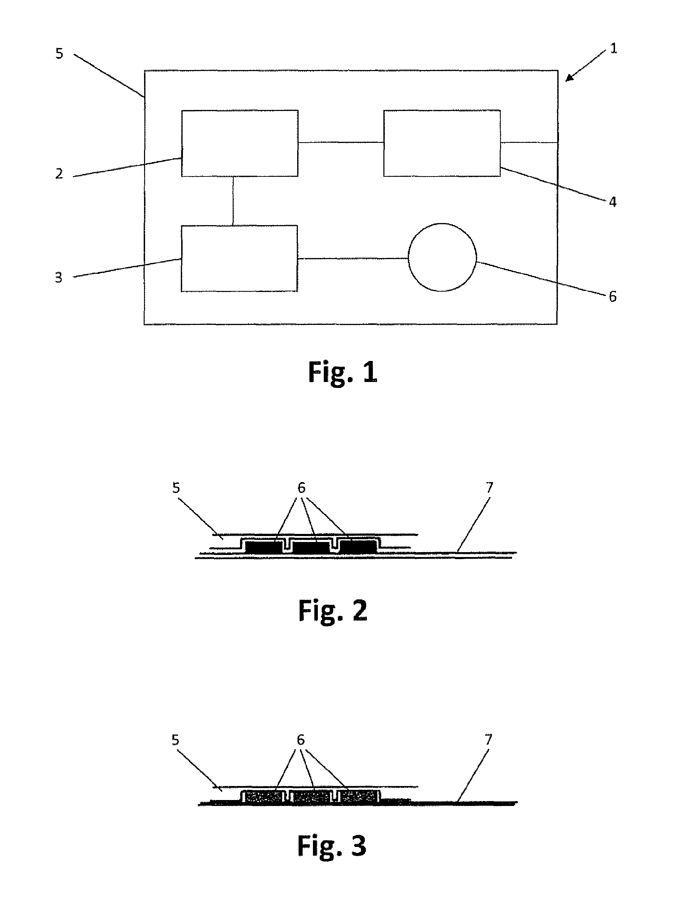

Figure 1 shows a systematic overview of an infusion device 1. The device consists of a housing 5 that houses a removable drug cartridge 4, a motor 2 and a control unit 3. The control unit 3 controls the motor 2 to move a piston within the drug cartridge 4. If the piston is moved forward, the drug held in the cartridge 4 is delivered to a patient via a catheter. Alternatively, the motor 2 may be the motor of a pump that moves the drug from the cartridge 4 into the catheter.

The control unit 3 also controls light emitting diodes 6 which indicate the state of the infusion device. The state can be made up of several sub-states like the administration rate of the device 1, the remaining content of the cartridge 4 or the remaining power of the battery (not shown) powering the infusion device 1.

Figure 2 shows one implementation of fixing the LEDs 6. The LEDs 6 are arranged in recesses of the housing 5 and are attached to a carrier 7. The carrier 7 is a circuit board that mechanically holds and electrically connects the LEDs 6. The housing 5 is transparent in the area covering the LEDs 6. There is a gap between the LEDs 6 and the housing 5

Figure 3 shows an alternative implementation in which the LEDs 6 are moulded into recesses of the housing 5. The circuit board 7 only electrically connects the LEDs 6. The housing 5 is transparent in the areas covering the LEDs 6.

In the drawings, the LEDs 6 are depicted as dots, circles, or rectangles. The symbol used in the drawings does not mean that the LEDs necessarily have the same shape as the symbol. The reference sign 6 indicates LEDs in general. Where an individualization of LEDs is necessary, single LEDs or groups of LEDs are marked with a reference sign consisting of the number 6 and a single character, for example 6a, 6b.

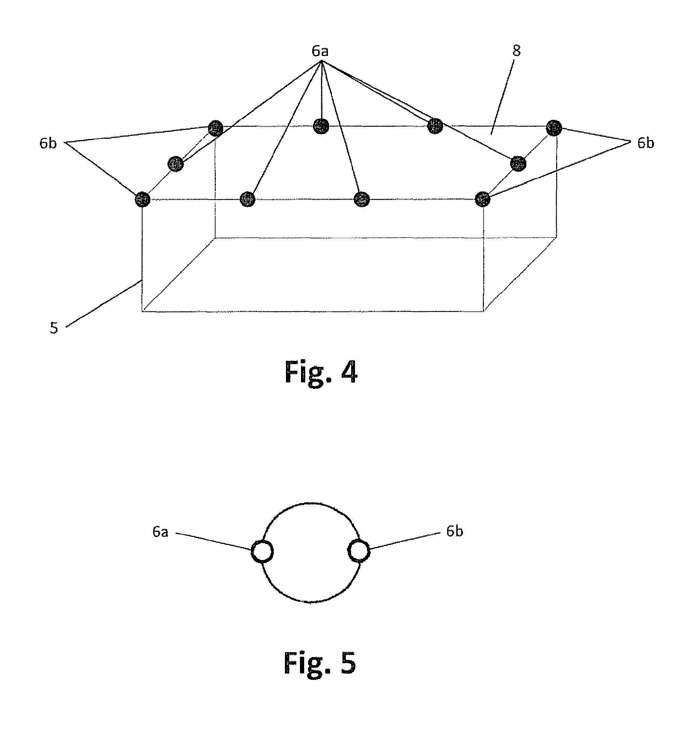

Figure 4 shows an exemplary housing 5 with a front plate 8. The front plate 8 holds the keys and buttons (not shown) used to operate the infusion device 1. The side of the housing 5 opposite to the front plate 8 usually faces the patient and holds fixing means for the infusion device 1.

Six LEDs 6a are arranged on the four edges of the front plate 8. In addition, 4 LEDs 6b are arranged on the corners of the front plate 8a. With this arrangement, the LEDs 6a and 6b are visible from a broad range of angles.

Referring to Figures 5 to 24, several arrangements of LEDs and indication modes for operating the LEDs according to the present invention are illustrated. Each indication mode comprises showing specific patterns on the arrangement of LEDs. It is possible to combine the arrangements and indication modes of two or more of the following embodiments.

The indication modes are based on a geometrical and/or temporal coding of the status, with a pattern or sequence of patterns indicating the status of the device 1. That means that a status or sub-status of the infusion device is coded in the pattern of illuminated LEDs, including the colour of each LED The temporal coding, that means a sequence of patterns, is used to show an activity of the infusion device 1 or to show several sub-states one after the other, for example using different colours.

The LEDs used in the following exemplary embodiments are multi-colour LEDs. A letter inside an LED indicates the colour of light the LED emits. The character r stands for red, the character g stands for green, the character b stands for blue and the character y stands for yellow. If there is no character inside an LED, the respective LED is off.

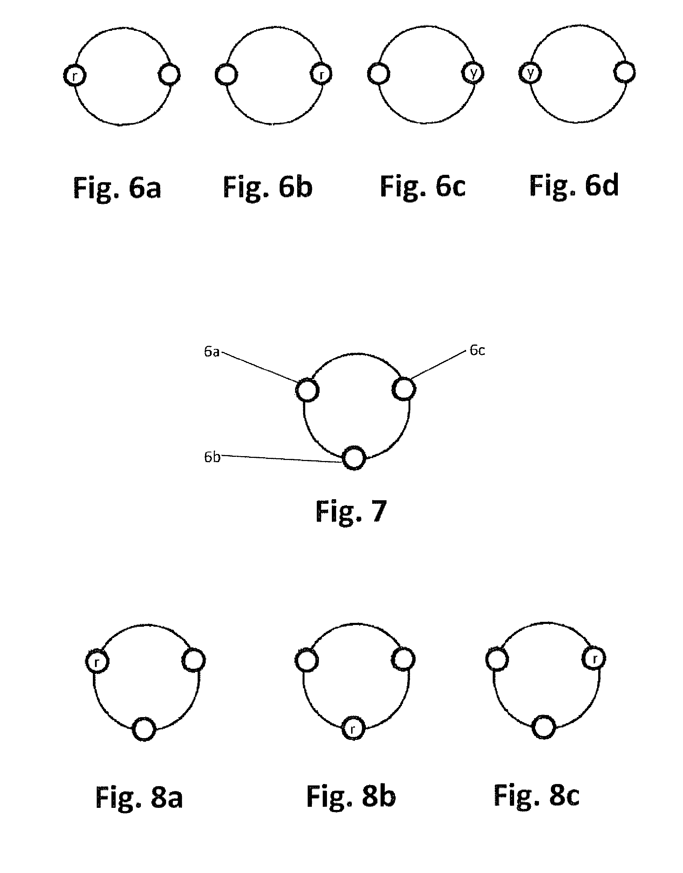

Figure 5 shows an arrangement of two LEDs 6a and 6b. An exemplary indication mode consists of the two patterns shown in Figures 6a and 6b. If the LEDs 6a (see Figure 6a) and 6b (see Figure 6b) are alternately shining red, it is indicated that the piston in the cartridge 4 is moved forward, that means the drug is administered to the patient. If the LEDs 6b (see Figure 6c) and 6a (see Figure 1d) are alternately shining yellow, the piston is moved backwards. The amount of time each LED 6a, 6b shines before the pattern changes is identical. Optionally, one LED shining longer than the other means the piston has reached an end position.

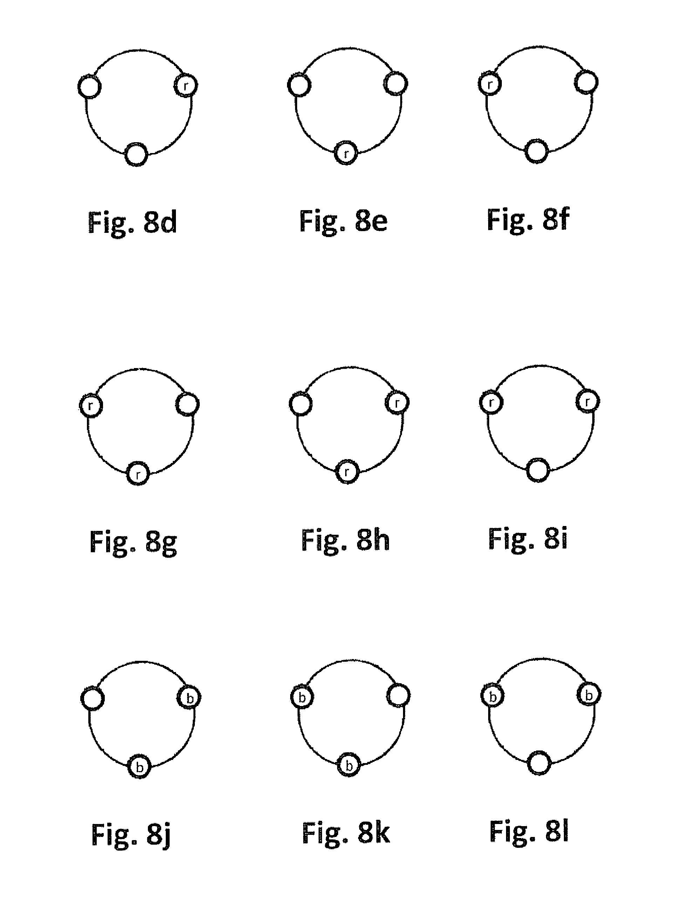

Shown in Figure 7 is a circular arrangement of three LEDs 6a, 6b and 6c. Figure 8 shows exemplary patterns of indication modes for this three LED arrangement. If the LEDs shine red in the order 6a, 6c, and 6b (as shown in the sequence of patterns depicted in Figures 8a, 8b and 8c), the piston slowly moves backwards. If the LEDs shine red in the order 6c, 6b and 6a (as shown in the sequence of patterns depicted in Figures 8d, 8e and 8f), the piston is moved forwards. If the patterns of Figure 8g, 8h and 8i are sequentially shown, resulting in a set of two neighbouring LEDs shining red and moving in a counter-clockwise circular movement, the piston is quickly moved backwards, for example prior to changing the cartridge 4.

In another indication mode, the patterns depicted in Figures 8j, 8k and 8l are sequentially shown. In each of the patterns, two of the LEDs shine blue. The temporal sequence of the patterns gives the impression of the LED that is turned off moving clockwise, or the set of two neighbouring bleu LEDs moving clockwise. This mode indicates a Bolus, which is a quick disbursement of the drug. If the drug is insulin, the Bolus is used, for example when the patient consumes a meal.

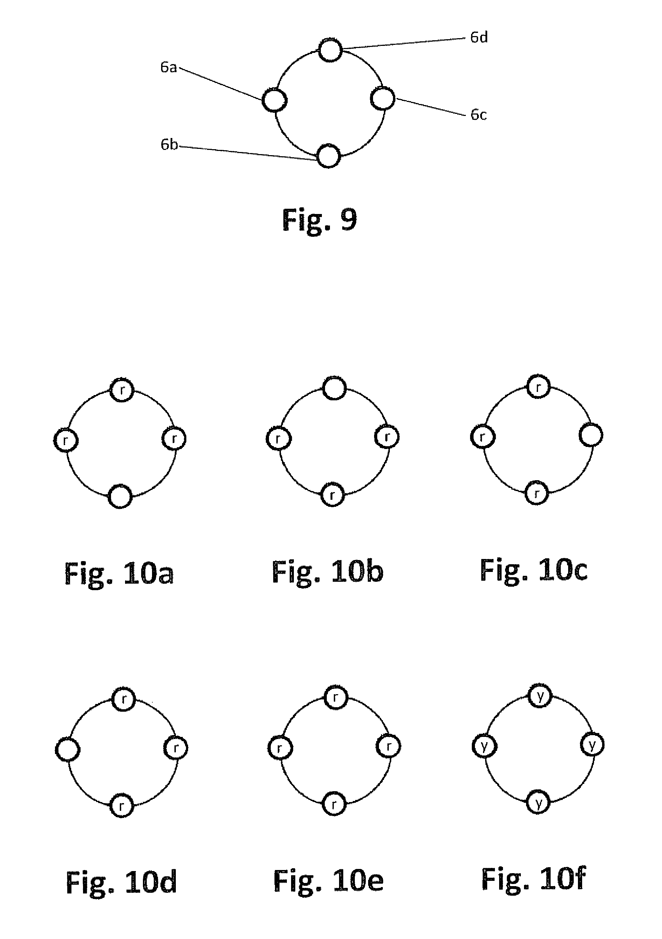

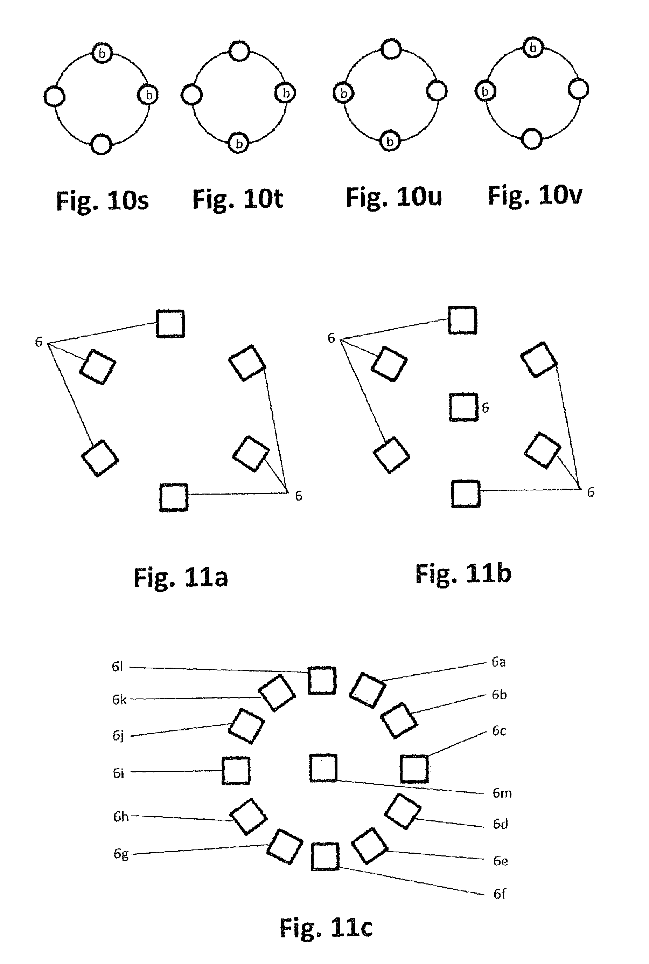

Figure 9 shows a circular arrangement of four LEDs 6a, 6b, 6c and 6d. Figure 10a shows a pattern in which LEDs 6a, 6c and 6d shine red. This indicates operation of an "up"-button (not shown). Similarly, the pattern shown in Figure 10b with LEDs 6a, 6b and 6c shining read indicates the operation of a "down"-button (not shown)". The pattern shown in Figure 10c with the LEDs 6a, 6b and 6c shining red indicates backwards movement of the piston.

If the pattern shown in Figure 10d, that is using LEDs 6b, 6c and 6d, flashes red, it is indicated that the cartridge 4 is empty. If the pattern shown in Figure 10e, that is using all four LEDS 6a, 6b, 6c and 6d, flashes, it is indicated that there is an occlusion. If the pattern of Figure 10f, that is all four LEDs 6a, 6b, 6c and 6d shining yellow, is shown, it is indicated that a failure is present.

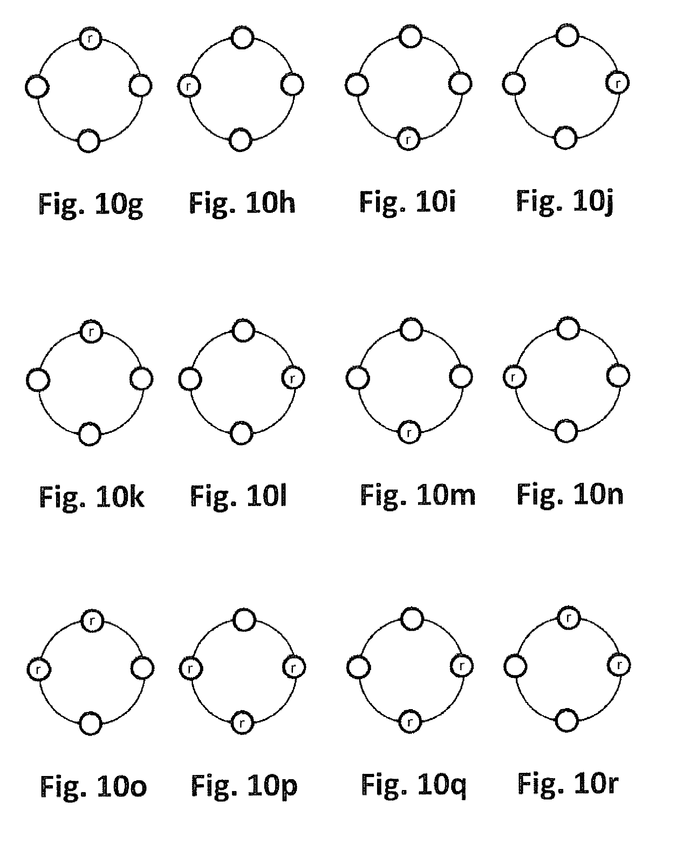

If the patterns illustrated in Figures 10g, 10h, 10i and 10j are shown sequentially, that is an LED shining red moves counter-clockwise, it is indicated that the piston is slowly moved backwards. If the sequence of the patterns of Figures 10k, 10l, 10m and 10n is shown, that is a red LED moving clockwise, it is indicated that the drug is slowly delivered, for example for filling the catheter between the infusion device 1 and the patient.

If the sequence of the patterns of Figures 10o, 10p, 10q and 10r is shown, that is two neighbouring red LEDs moving counter-clockwise, it is indicated that the piston is quickly moving backwards.

If the sequence of the patterns in Figures 10s, 10t, 10o and 10v is shown, that is two neighbouring blue LEDs moving clockwise, disbursement of a Bolus is indicated.

Figure 11 a shows an embodiment with 6 LEDs 6 arranged in a circle. Figure 11 b shows an arrangement of 7 LEDs 6 that equals the arrangement of Figure 11a with an additional LED in the centre of the circle.

Figure 11c shows an arrangement of 12 LEDs 6a to 6l arranged in a circle where the LED 6a is at the one o'clock position and the LED 6l is at the 12 o'clock position. An optional thirteenth LED 6m is placed in the middle of the circle.

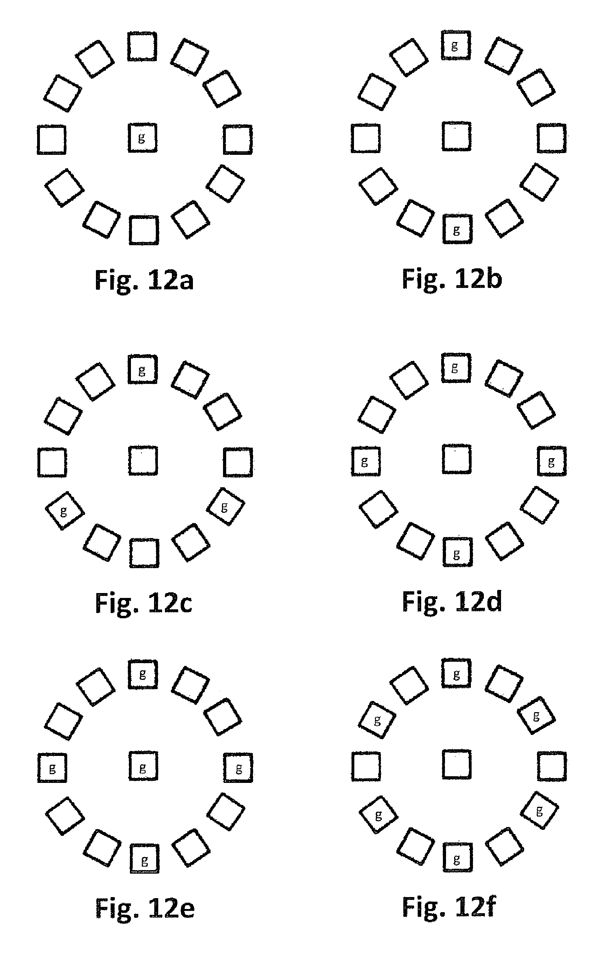

Shown in Figures 12a to 12m are exemplary patterns of a symmetrical indication mode using the arrangement of Figure 11c. This mode can be used for indicating an amount, for example of remaining battery power, remaining drug or delivery rate. Each pattern has a different number of active LEDs. In the present example, an active LED means that the LED is shining green.

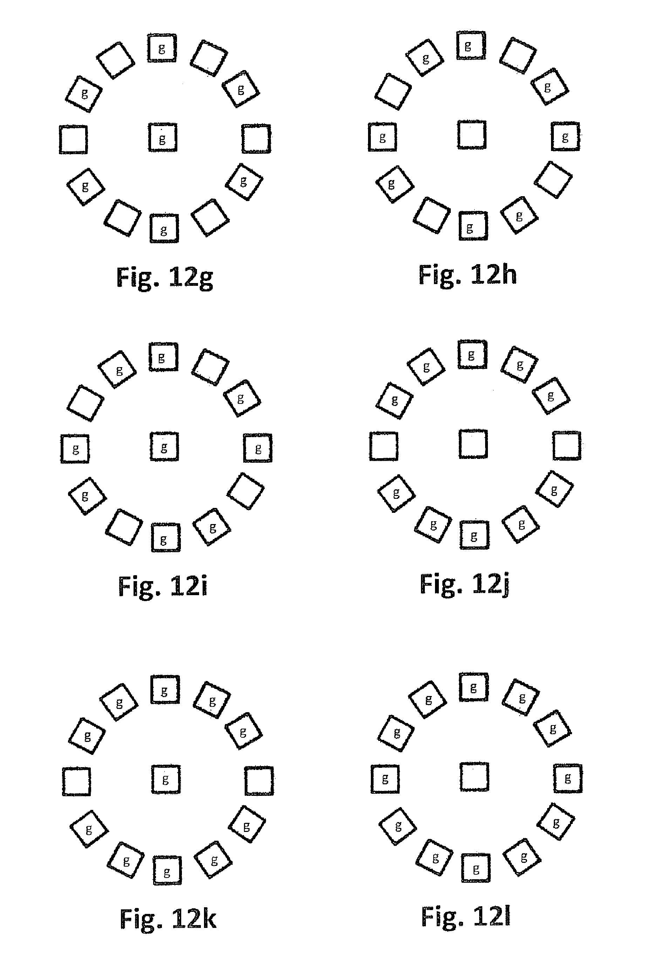

In the pattern shown in Figure 12a, only the central LED 6m is active. In the pattern of Figure 12b, the two LEDs 6f and 6l are active. In the pattern shown in Figure 12c, the three LEDs 6d, 6h and 6l are active. In the pattern shown in Figure 12d, the four LEDs 6c, 6f, 6i and 6l are active. In the pattern of Figure 12e, the five LEDs 6c, 6f, 6i, 6l and 6m are active. In the pattern shown in Figure 12f, the six LEDs 6b, 6d, 6f, 6h, 6j and 6l are active. In the pattern shown in Figure 12g, the seven LEDs 6b, 6d, 6f, 6h, 6j, 6l and 6m are active.

In the pattern shown in Figure 12h, the eight LEDs 6b, 6c, 6e, 6f, 6h, 6i, 6k and 6l are active. In the pattern shown in Figure 12i, the nine LEDs 6b, 6c, 6e, 6f, 6h, 6i, 6k, 6l and 6m are active. In the pattern shown in Figure 12j, the ten LEDs 6a, 6b, 6d to 6h and 6j to 6l are active.

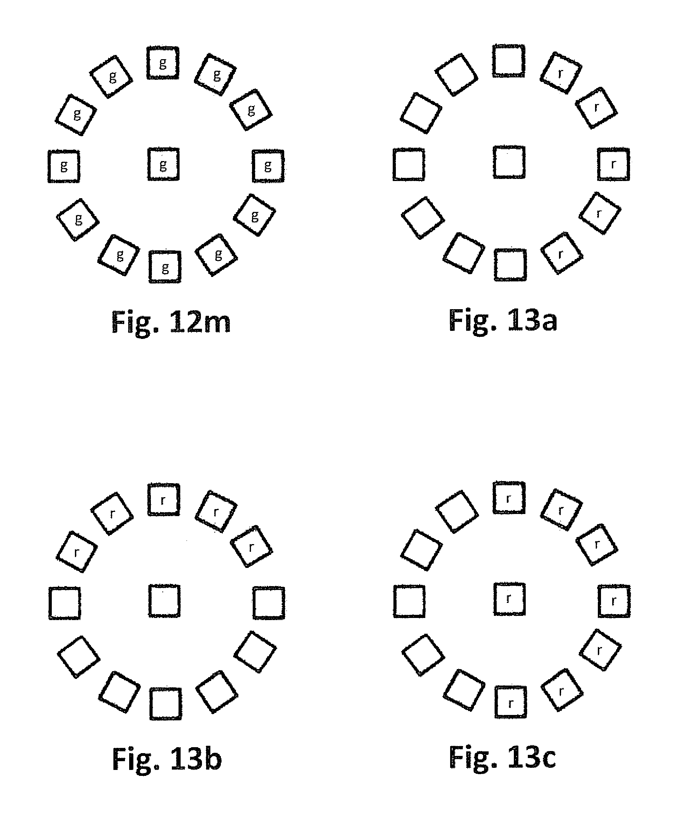

In the pattern shown in Figure 12k, the eleven LEDs 6a, 6b, 6d to 6h, 6j to 6l and 6m are active. In the pattern shown in Figure 12l, the twelve LEDs 6a to 6l are active. If the patterns of Figures 12a to 12l are shown sequentially, the circle of LEDs is gradually filling. In the pattern shown in Figure 12m, all thirteen LEDs 6a to 6l and 6m are active. The indication mode using the patterns according to Figures 12a to 12m allows for a quick visible recognition of the amount that is indicated.

If the pattern shown in Figure 13a, that is of red LEDs 6a to 6e, is flashing, the piston is slowly moved forwards, for example for filling the catheter. If the pattern of Figure 13b is shown, that is LEDs 6a, 6b and 6j to 6l shining red, it is indicated that the "up"-button is pressed. If the pattern of Figure 13c is shown, that is LEDs 6a to 6f, 6l and 6m shining red, it is indicated that the cartridge 4 is empty.

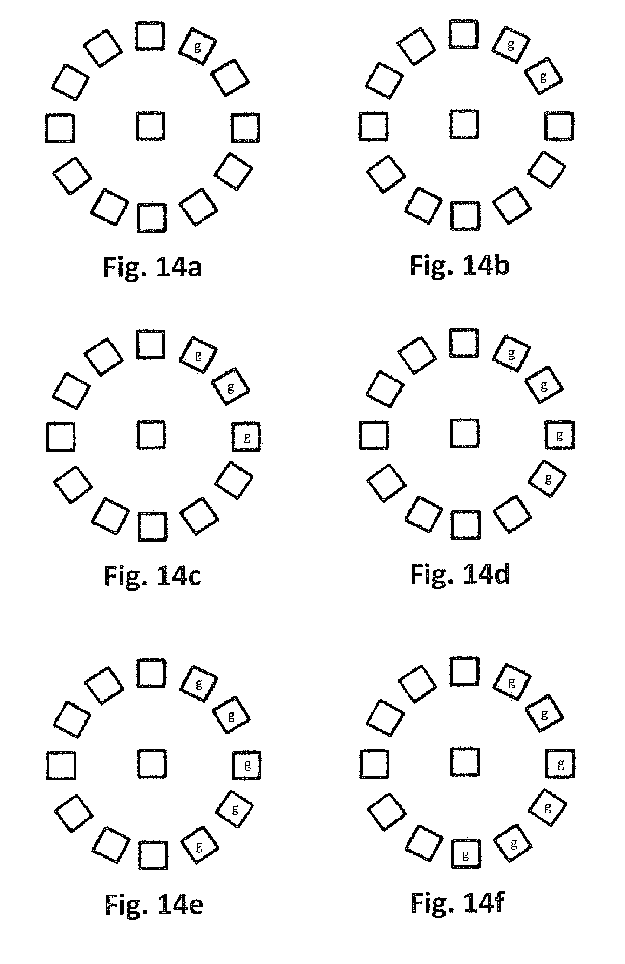

The patterns depicted in Figures 14a to 14m show clock-like patterns as an alternative to the patterns shown in Figures 13a to 13m. If the sequence of the patterns of Figures 14a to 14m is shown, the number of active LEDs is linearly increasing.

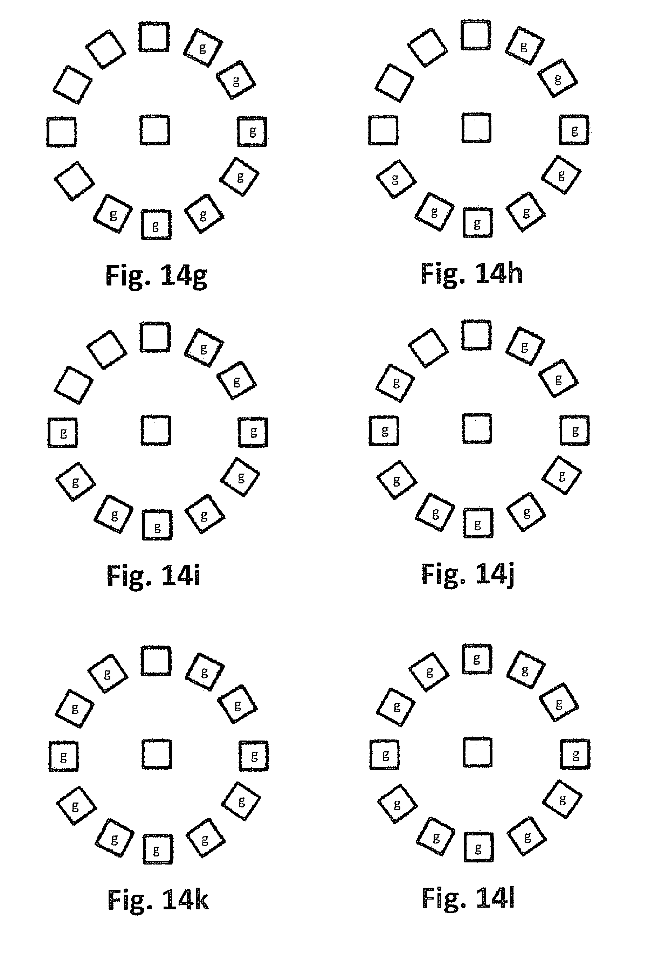

In the pattern of Figure 14a, only one LED 6a is active. An active LED is example means the LED is shining green. In the pattern of Figure 14b, the two LEDs 6a and 6b are active In the Pattern of Figure 14c, the three LEDs 6a to 6c are active. In the Pattern of Figure 14d, the four LEDs 6a to 6d are active. In the Pattern of Figure 14e, the five LEDs 6a to 6e are active. In the pattern of Figure 14f, the six LEDs 6a to 6f are active. In the pattern of Figure 14g, the seven LEDs 6a to 6g are active. In the pattern of Figure 14h, the eight LEDs 6a to 6h are active.

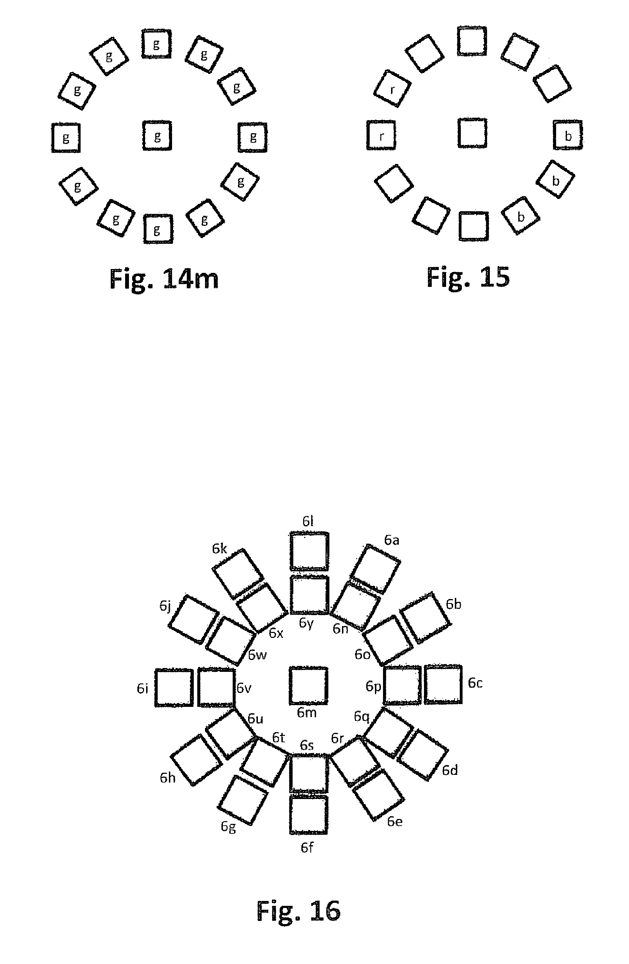

In the pattern of Figure 14i, the nine LEDs 6a to 6i are active. In the pattern of Figure 14j, the ten LEDs 6a to 6j are active. In the pattern of Figure 14k, the eleven LEDs 6a to 6k are active. In the pattern of Figure 14l, the twelve LEDs 6a to 6l are active. In the pattern of Figure 14m, the thirteen LEDs 6a to 6m are active.

The arrangement of Figure 11c can also be used to display two or more sub-states at the same time. In the example according to Figure 15, the six LEDs 6c to 6h are used to indicate the remaining drug in the cartridge using blue colour and the six LEDs 6i to 6l, 6a and 6b are used to indicate the remaining battery power in red colour In the exemplary pattern shown in Figure 15, the cartridge is half full (three of the six LEDs shining blue) and the battery has one third of its capacity left (two of the six LEDs shining red).

Shown in Figure 16 is an arrangement of twenty-five LEDs 6a to 6y. LEDs 6a to 6l form an outer ring and LEDs 6n to 6y form an inner ring which is concentric to the outer ring. In the centre of both rings, the LED 6m is located.

The centre LED 6m can be used to indicate whether an error (red colour) or an alert (yellow colour) has occurred. The other LEDs can be used to indicate the number of the error or the alert.

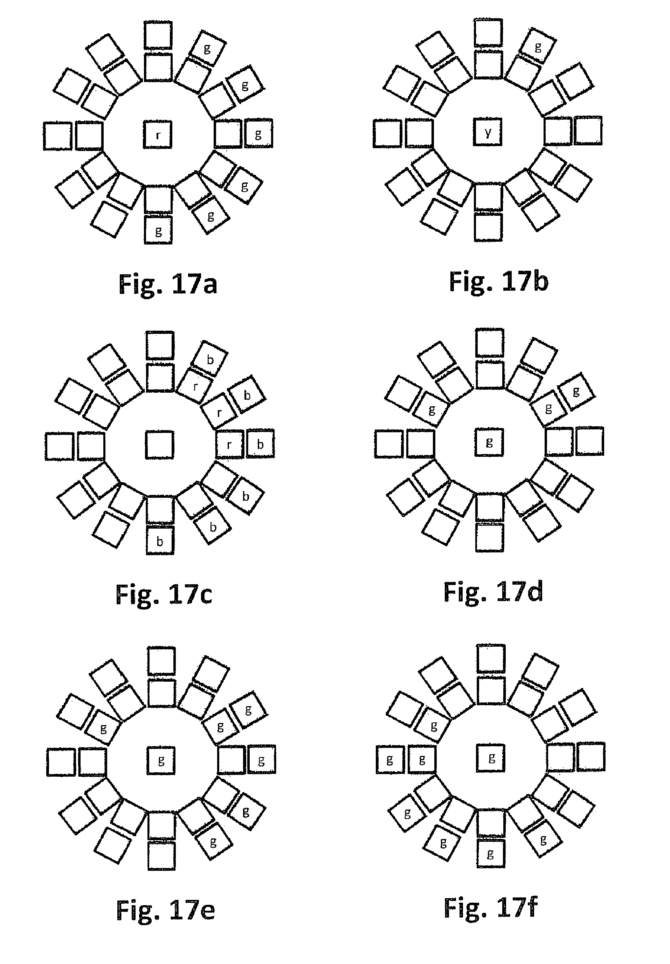

In the pattern shown in Figure 17a, the central LED 6m flashes red and the six LEDs 6a to 6f of the outer ring shine green. The red flashing LED 6m indicates that an error has occurred, and the six LEDs 6a to 6f indicate that the error has error number 6.

In the pattern shown in Figure 17b, the central LED 6m quickly flashes twice in yellow and the LED 6a continuously shines green. The yellow flashing LED 6m indicates that an alert has occurred and the green LED 6a indicates that the alert has alert number 1. Alternatively, the LED 6m continuously shines yellow without flashing.

Figure 17c shows a pattern of another indication mode with the remaining cartridge content indicated in blue by the outer ring LEDs 6a to 61 and the remaining battery capacity indicated red by the inner ring LEDs 6n to 6y. In the exemplary status shown in the pattern of Figure 17c, six of the twelve outer ring LEDs are shining blue, which means that the cartridge is half full. The three LEDs 6n to 6p of the twelve LEDs constituting the inner ring are shining red, thus indicating that the remaining battery capacity is one fourth.

The arrangement of Figure 16 can also be used for indicating the time. One possibility is to emulate an analogous clock. This is shown in the exemplary pattern of Figure 17d. The central LED 6m shining green indicates the clock mode. One LED of the inner ring indicates the hour while the combination of an LED in the inner ring and a corresponding, adjacent LED of the outer ring represents the minute.

In the pattern shown in Figure 17d, the LED 6w indicates ten hours and the LEDs 6o and 6b indicate 10 minutes. This mode offers a five minutes accuracy.

Another possibility is additionally using LEDs of the outer ring to gain a one minute accuracy. The number of additionally shining LEDs in the outer ring indicates the number of minutes passed since the last full five minutes. The extra minute indicating LEDs are preferably extending clockwise from the outer ring LED resembling the minute pointer of the clock.

In the example shown in Figure 17e, the LED 6w indicates 10 hours, the LEDs 6o and 6b indicate 10 minutes and the LEDs 6c, 6d and 6e indicate additional three minutes, resulting in 10:13.

The indication mode shown referring to Figure 17f is similar to that shown in Figure 17e, with the difference that the additional minutes are shown by outer ring LEDs extending counter-clockwise from the outer ring LED resembling the minute pointer. In the example shown in Figure 17f, the LED 6w indicates 10 hours the LEDs 6v and 6i indicate 45 minutes. The four LEDs 6e to 6h indicate additional four minutes, thus resulting in 10:49. Another possibility is the outer LED of the LEDs which resemble the minute pointer flashing a number of times, corresponding to the number of additional minutes.

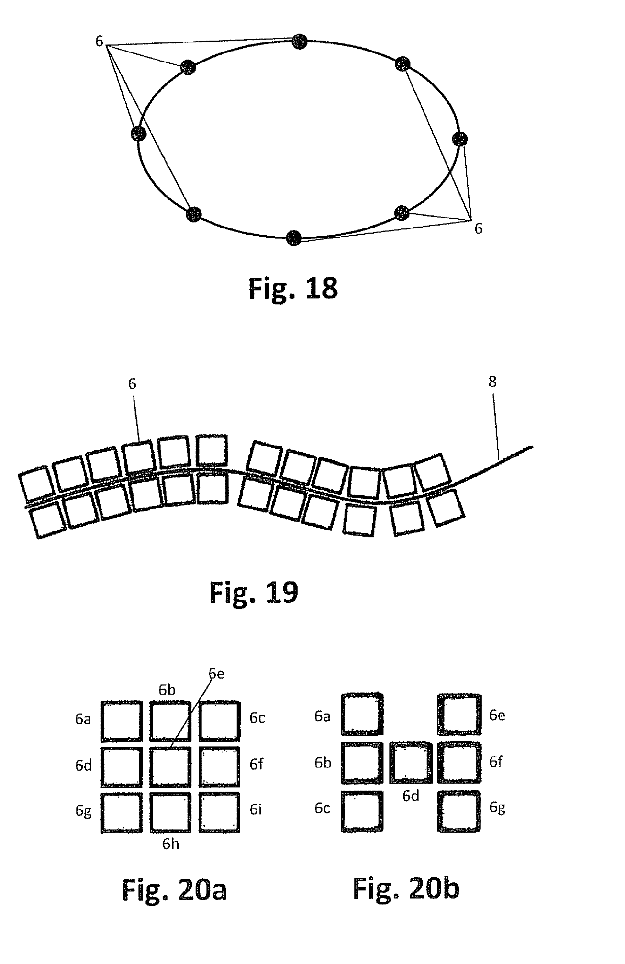

Figure 18 shows another arrangement of eight LEDs 6 arranged on an ellipse. The distance between pairs of neighbouring LEDs along the ellipse may be constant or varying. With this arrangement, the indication modes explained with reference to Figures 10a to 10v, 13a, 13b and 14a to 14l can be implemented, of course adapted to the number of LEDs arranged on the ellipse. Optionally, there can be at least one LED within the ellipse, for example in the centre of the ellipse, so the indication modes explained with reference to Figures 12a to 12m, 13c, 14m and 15 can be implemented, of course adapted to the number of LEDs arranged on the ellipse. Similarly, there may be two ellipses with one ellipse within the other ellipse, so the indication modes explained with reference to Figures 17a to 17f can be implemented, of course adapted to the number of LEDs arranged on the ellipses. In the latter case, too, one or more LEDs may be arranged within the inner ellipse.

Figure 19 shows another alternative arrangement of twenty-four LEDs 6 (of which only one is marked with a reference sign) along a path 8. The path 8 may have any suitable shape, for example depending on the shape of the housing 5. Each line of LEDs, of which there are two parallel ones in Figure 19, can be used to show a bar chart with a sub-status of the infusion device 1.

Figure 20 shows two arrangements of LEDs resembling a dice. The arrangement shown in Figure 20a is a 3x3 matrix with nine LEDs 6a to 6i. The arrangement shown in Figure 20b consists of two parallel columns with three LEDs each (6a to 6c and 6e to 6g) and a seventh LED 6d arranged between the central LEDs 6b and 6f of the two columns. Those arrangements can display numbers in a dice-like manner.

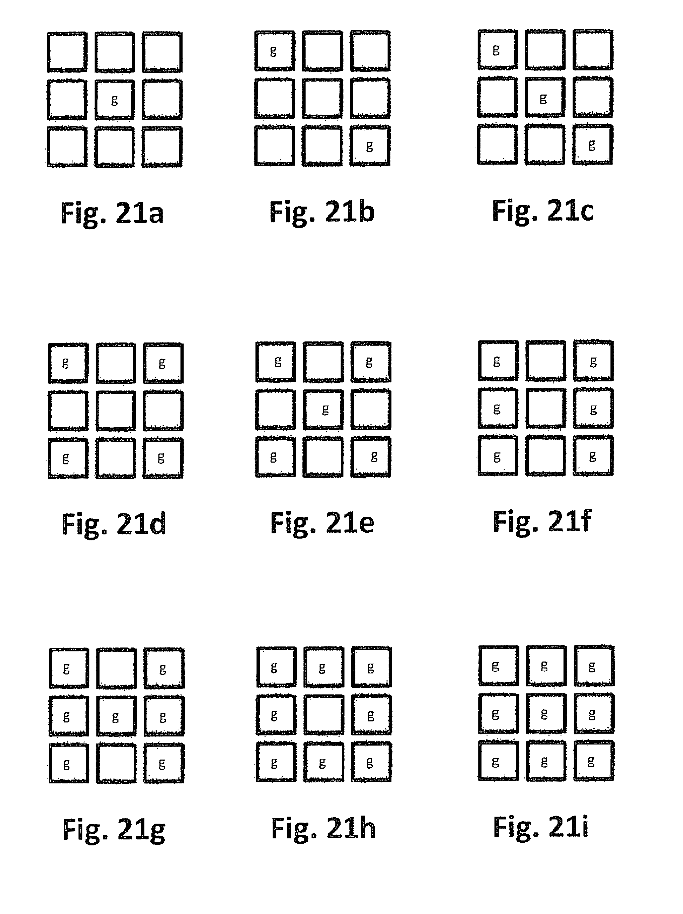

Patterns of an indication mode using the arrangement of Figure 20a are shown in Figures 21a to 21i.

In the pattern shown in Figure 21a, the central LED 6e is shining green, thus indicating the absolute value one. In the pattern shown in Figure 21b, the LEDs 6a and 6i are shining green, thus indicating the absolute value 2. In the pattern shown in Figure 21c, the LEDs 6a, 6e and 6i are shining green, thus indicating the absolute value three. In the pattern shown in Figure 21d, the LEDs 6a, 6c, 6g and 6i are shining green, thus indicating the absolute value four.

In the pattern shown in Figure 21e, the LEDs 6a, 6c, 6e, 6g and 6i are shining green, thus indicating the absolute value five. In the pattern shown in Figure 21f, the LEDs 6a, 6c, 6d, 6f, 6g and 6i are shining green, thus indicating the absolute value six. In the pattern shown in Figure 21g, the LEDs 6a, 6c, 6d, 6e, 6f, 6g and 6i are shining green, thus indicating the absolute value seven. In the pattern shown in Figure 21h, the LEDs 6a to 6c, 6d, 6f and 6g to 6i are shining green, thus indicating the absolute value eight. In the pattern shown in Figure 21i, the LEDs 6a to 6i are shining green, thus indicating the absolute value nine.

With the arrangement of Figure 20b, the patterns depicted in Figures 21a to 21g can be shown, of course adapting the denomination of the LEDs according to the different reference signs used in Figures 20a and 20b.

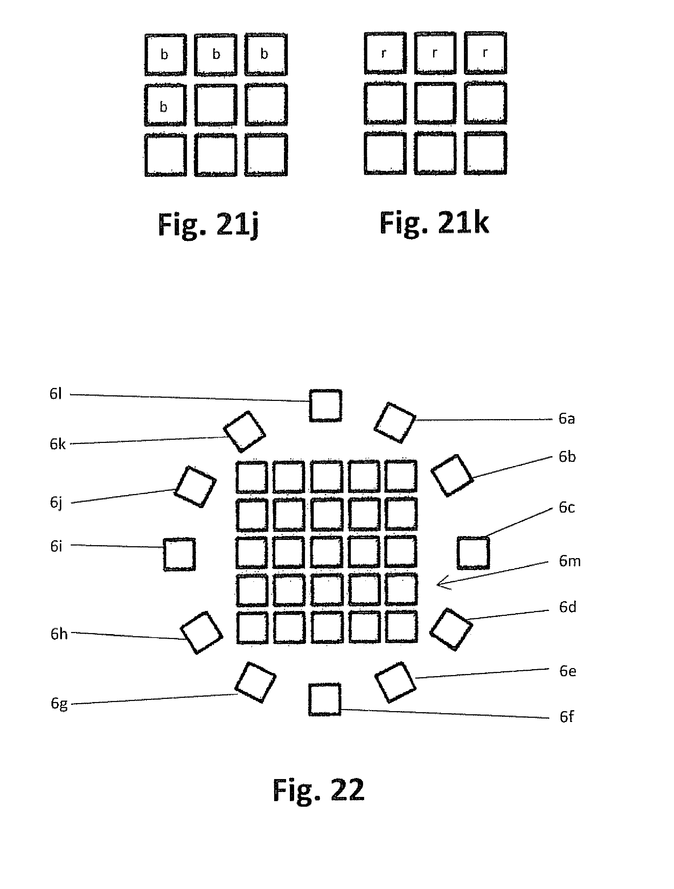

Figure 21j shows an exemplary pattern of a mode indicating the remaining drug in the cartridge 4. The number of LEDs shining blue indicates the quota of remaining drug. In the pattern shown in Figure 21j, four LEDs 6a to 6d out of the nine LEDs 6a to 6i shine blue, thus indicating a fill level of four ninth of the cartridge 4.

The battery charge indication mode is explained referring to an exemplary pattern shown in Figure 21k, indicating the battery charging level by an amount of LEDs shining red. In the pattern shown in Figure 21k, the three LEDs 6a to 6c out of the nine LEDs 6a to 6i shine red, thus indicating a remaining battery capacity of one third.

In the indication mode using the patterns according to Figures 21j and 21k, the remaining drug in the cartridge 4 and the remaining capacity of the battery, respectively, is shown by the quota of shining LEDs. The matrix of LEDs is filled row by row and from the left to right within each row.

Figure 22 shows an arrangement of thirty-seven LEDs 6a to 6m. Twelve LEDs 6a to 6l are arranged in a circle. Twenty-five LEDs, collectively denoted 6m, are arranged in a 5x5 matrix inside the circle formed by LEDs 6a to 6l. The circle of LEDs 6a to 6l can be used to indicate an absolute value, for example as described with regard to Figures 14a to 14l. The matrix of LEDs 6m can be used to display a digit, a character or any information coded in another way. One example for this coding is the same as described with regards to Figures 21j and 21k, but scaled from a 3x3 matrix to a 5x5 matrix. Another possibility is to always activate every LED of a row and code the information in the number of activated rows. Preferably only neighbouring rows are activated, beginning with the top row.

Instead of the matrix of LEDs 6m, a bar of LEDs or a symbol can be arranged within the circle of LEDs.

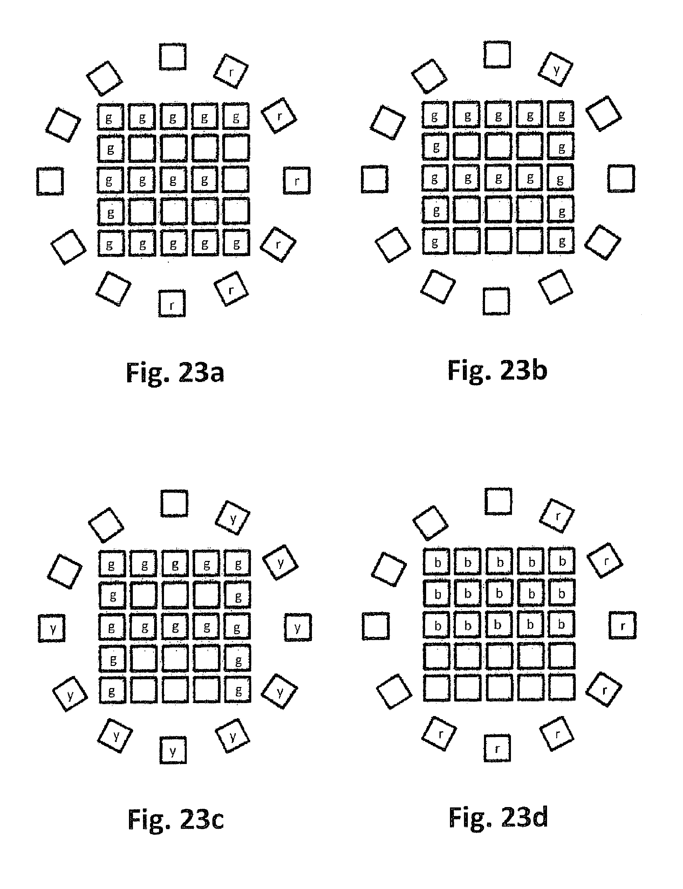

One indication mode is explained referring to the patterns of Figures 23a to 23c. In this indication mode, the matrix of LEDs 6m is used to the display a character, for example in green colour. The characters used in this embodiment are an E for indicating an error and an A for indicating an alert. The circle of LEDs 6a to 6l is used to indicate the number of the error or alert.

An alert is given to the user mainly for information purposes, for example to indicate that the drug cartridge 4 will be empty in a few hours. However, an alert is no indication of a dangerous situation and does not need immediate reaction. In contrast, an error requires immediate action and is given for a blocked infusion line, a fully exhausted drug cartridge 4 or a device fault, for example.

In the pattern shown in Figure 23a, the matrix of LEDs 6m displays an E and the six LEDs 6a to 6f of the circle shine red. This pattern indicates that error number six is present.

If the matrix 6m of LEDs shows an A, the alert number is indicated by the circle LEDs shining yellow and not red as for an error. Using the colour of the LEDs 6a to 6l as well as the characters shown by the matrix LEDs 6m is a redundancy that allows for a save distinction between error and alerts.

In the pattern shown in Figure 23b, the matrix 6m of LEDs displays an A. Only one LED 6a of the circle of LEDs shines yellow, thus indicating the value one. This means, that the pattern shown in Figure 23b indicates alert number one.

In the pattern shown in Figure 23c, the matrix of LED 6m alternately displays an A or an E and a number. The number indicates the alert or error number. In this exemplary pattern, the matrix of LEDs 6m alternately displays an A and the number 6, thus indicating alert number 6. The LEDs 6a to 6i of the circle are flashing yellow, with the number of flashing LEDs corresponding to the importance of the alert (or error) and/or the time until an action is ultimately required, for example replacing the drug cartridge 4. Important alerts or errors may be any alerts or errors with number 5 or higher, for example.

Another indication mode is explained referring to an exemplary pattern shown in Figure 23d. The matrix of LEDs 6m is used to indicate the remaining drug in blue colour and the circle LEDs 6a to 6l are used to indicate the remaining battery capacity in red colour The quota of remaining drug or battery capacity is indicated by the number of shining LEDs in the circle and the number of shining rows in the matrix, using the same principle as in the indication modes explained referring to the patterns shown in Figures 14a to 14l for the circle LEDs and Figures 21a to 21k for the matrix LEDs.

In the pattern shown in Figure 23d, the first three rows of the matrix are shining blue, thus indicating a remaining amount of drug of three fifth. The seven LEDs 6a to 6g shine red, thus indicating a remaining battery capacity of seven twelfth.

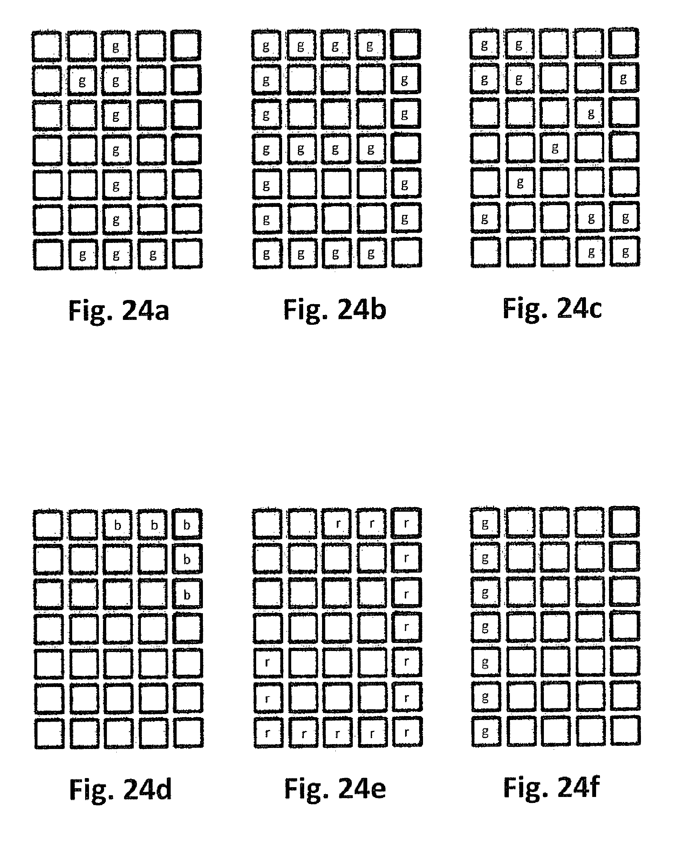

Another arrangement of the LEDs is a matrix, for example a 5x7 matrix. The matrix may be used to display characters, digits or special characters. In the pattern shown in Figure 24a, the LEDs are displaying the digit 1. In the pattern shown in Figure 24b, the LEDs are displaying the character B, indicating a Bolus being disbursed. In the pattern shown in Figure 24c, the LEDs are displaying a percent sign.

Numbers consisting of more than one digit or combinations of more than one character can be displayed by scrolling the text over the matrix, for example from right to left, or by displaying the digits and characters one after the other. Each digit or character may be displayed for one second with a pause of 0.2 seconds between consecutive characters or digits.

Another indication mode explained referring to a pattern shown in Figure 24d shows the quota of remaining drug in the cartridge 4. The outer LEDs of the matrix are used to indicate the quota in blue colour by the quota of shining LEDs in a clock-like manner, starting with the central LED in the uppermost row. In the exemplary pattern shown in Figure 24d, five of the twenty border LEDs are shining blue, thus indicating a quota of 25% of drug remaining.

An indication mode for indicating a remaining battery capacity is similar to the mode described for indicating the remaining drug, with the difference that the LEDs are shining red. In the exemplary pattern shown in Figure 24e, fifteen of the twenty border LEDs are shining red, thus indicating a remaining battery capacity of 75%.

An indication mode for indication filling of the catheter is explained referring to the pattern shown in Figure 24f. In this mode, a row of shining LEDs is moving from left to right to indicate that the catheter is filled with the drug, which means the piston is moving forwards in the cartridge 4. Figure 24f shows the first pattern of this sequence of patterns, with the LEDs of the leftmost column of the matrix shining green.

It is possible to use the matrix of LEDs to display the remaining drug as well as the remaining battery capacity one of the other. Another possibility is to indicate both information at the same time. For example, the 5x7 matrix of LEDs can be divided into a central 3x5 matrix that is surrounded by the border LEDs of the 5x7 matrix, displaying different information using the 3x5 matrix and the border LEDs. The concept for indicating the information using the 3x5 matrix is basically the same as described for the 5x7 matrix, with the difference that there are only nine border pixels which can be used.

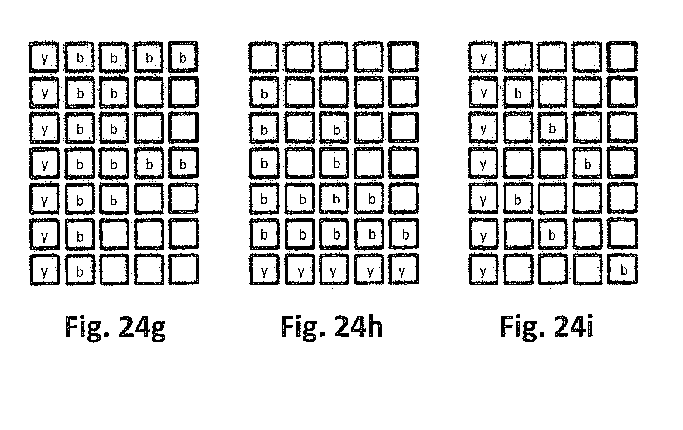

Another indication mode uses the matrix of LEDs to display a bar chart. Exemplary patterns of this indication mode are depicted in Figures 24g to 24i. In this indication mode, one complete row or complete column is shining in a first colour to identify the baseline of the bar chart. In the patterns shown in Figures 24g to 24i, yellow colour is used to identify the baseline. In the patterns shown in Figures 24g and 24i, the leftmost column is used as a baseline with the bars extending to the right, while in the pattern shown in Figure 24h, the lowermost row is used as a baseline with the bars extending upwards.

The bars are displayed in a second colour; in the exemplary pattern shown in Figures 24g to 24i, this second colour is blue. The bars can be displayed fully or only the LED determining the peak of the bar can shine. Each bar indicates a sub-status of the infusion device 1.

The matrix may have any other size, for example 10 by 7 to display two characters or digits and the same time.

The indication modes explained referring to figures 10a to 10v, 13a, 13b, 14a to 14l and 15 can be implemented using the arrangement shown in Figure 4, of course adapted to the number of LEDs arranged on the corners and/or edges of the front plate.

It is possible to implement all indication modes with the patterns flashing instead of shining continuously, which causes more attention of the patient and might make recognition of the state of the device easier. Another possibility is to indicate that an error or alert is present and to identify the error or alert after a key or button is pressed.

In the arrangements shown in the Figures 7, 9, 11a, 11b, 11c, 16 and 22, the LEDs may be arranged on a segment of a circle instead of on a full circle.

## Claims

1. Infusion device (1) with a housing (5), an actuator (2) and a control unit (3), **characterized by** a plurality of optical indicators (6) in a defined geometrical arrangement for indicating the state of the infusion pump device (1).

2. Infusion device (1) according to claim 1, **characterized in that** the optical indicators are at least in part LEDs (6).

3. Infusion device (1) according to claim 2, **characterized in that** the LEDs (6) are at least in part SMDs.

4. Infusion device (1) according to one of claims 1 to 3, **characterized in that** the housing (5) is at least partially transparent.

5. Infusion device (1) according to one of claims 1 to 4, **characterized in that** the optical indicators (6) are at least in part multi-color optical indicators (6).

6. Infusion device (1) according to one of claims 1 to 5, **characterized in that** the optical indicators (6) are at least in part located on the edges and/or corners of the housing (5).

7. Infusion device (1) according to claim 6, **characterized in that** at least one optical indicator (6) is placed on at least one or a plurality of or each edge and/or at least one or a plurality of corners of the housing (5) or at least one plate (8) of the housing (5).

8. Infusion device (1) according to one of claims 1 to 5, **characterized in that** at least some of the optical indicators (6) are arranged in or on an ellipse.

9. Infusion device (1) according to claim 8, **characterized in that** at least some of the optical indicators (6) are arranged in or on two or more concentric ellipses.

10. Infusion device (1) according to one of claims 8 or 9, **characterized by** at least five optical indicators (6) per ellipse.

11. Infusion (1) device according to one of claims 8 to 10, **characterized by** an optical indicator (6) arranged in the center of the ellipse or ellipses.

12. Infusion device (1) according to one of claims 8 to 11, **characterized in that** the ellipse is a circle.

13. Infusion device (1) according to one of claims 1 to 12, **characterized in that** at least some of the optical indicators (6) are arranged in a matrix.
